# EUROPEAN PATENT APPLICATION

(11) **EP 2 345 630 A2**
(43) Date of publication of application: **20.07.2011**
(21) Application number: 09813218.6
(22) Date of filing: 04.09.2009
(51) Int. Cl.: C07C 43/03

(54) **PLASTICISER AND POLYVINYL CHLORIDE (PVC) RESIN CONTAINING THE SAME**

(30) Priority: 12.09.2008 KR 20080090477
(71) Applicant: SK Innovation Co. Ltd., Jongno-gu Seoul 110-110 (KR); SK Global Chemical, Seoul 110-110 (KR)
(72) Inventor: HONG, Seung Gweon, Daejeon 305-728 (KR); KWON, Tae Wook, Daejeon 305-761 (KR); WANG, Hao, Seoul 110-110 (KR); KOH, Jae Suk, Daejeon 305-370 (KR)
(74) Representative: Noel, Chantal Odile
(86) International application number: PCT/KR2009/005022
(87) International publication number: WO 2010/030096

(57) **Abstract**

The present invention provides an ester plasticizer, particularly, a plasticizer for a polyvinyl chloride (PVC) resin, and a plastic comprising the plasticizer. More concretely, the present invention provides a plasticizer prepared by the esterification reaction of glycerol and organic acid.

When a polyvinyl chloride resin composition is manufactured using the ester plasticizer of the present invention, there are advantages in that a product having excellent plasticizing efficiency can be obtained, and in that the physical properties such as tensile strength and the like of the product are improved.

## Description

### Technical Field

The present invention relates to an ester compound and a plastic comprising the same, and, particularly, to a plasticizer for a polyvinyl chloride (PVC) resin. More precisely, the present invention relates to an ester plasticizer prepared using the esterification reaction of glycerol and carboxylic acid, by which a polyvinyl chloride resin composition having high plasticizing efficiency and improved physical properties such as hardness, tensile strength and the like can be manufactured.

### Background Art

A polyvinyl chloride resin, which is a homopolymer or a copolymer including 50% or more of polyvinyl chloride, is a commonly-used resin which can be formed into various products by extrusion molding, injection molding, calendering or the like. Such a polyvinyl chloride resin is widely used as a raw material for various products, such as pipes, electric wires, electric appliances, toys, films, sheets, synthetic leathers, tarpaulins, tape, packing materials for foods, medical supplies and the like. Various physical properties can be provided to such a polyvinyl chloride resin by suitably adding various additives such as a plasticizer, a stabilizer, filler, pigment and the like.

Among such additives, a plasticizer is an essential additive providing various physical properties and functions, such as workability, flexibility, electrical insulation, adhesivity and the like, to a polyvinyl chloride resin. Low volatility, as a very important factor of a plasticizer, is important both when a plasticizer is mixed in a plastic composition and when a shaped product containing a plasticizer is practically used. Further, plasticizers used in the field of foods, drinks, medical supplies and medicines must be harmless to the human body for health. A typical example of such harmless plasticizers is a phthalate plasticizer. However, it is predicted that the usage of a phthalate plasticizer will be remarkably reduced in the future because of the criticism regarding its toxicity attributable to its reactivation under the laws regulating toxic materials. Therefore, it is required to develop a plasticizer that includes an ester whose basic structure does not contain phthalate and which has a plasticizing efficiency equal to that of a phthalate plasticizer.

### Disclosure

### Technical Problem

In order to solve the above-mentioned problem, the present inventors carefully examined ester compounds that could be used as a plasticizer for a polyvinyl chloride resin. As a result, they found that a specifically-structured novel ester compound derived from glycerol can be used as a plasticizer, and, particularly, is excellent as a plasticizer for a polyvinyl chloride resin. Based on these findings, the present invention was completed. Accordingly, an object of the present invention is to provide a novel ester plasticizer prepared using glycerol and having physical properties equal to or superior to conventional phthalate plasticizers.

Another object of the present invention is to provide a plasticizer composition comprising the novel ester plasticizer.

Still another object of the present invention is to provide a polyvinyl chloride resin composition comprising the plasticizer composition.

### Technical Solution

In order to accomplish the above objects, a first aspect of the present invention provides an ester plasticizer, represented by Formula 1 below: 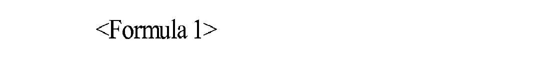 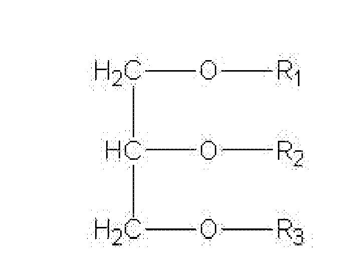 wherein R₁, R₂ and R₃ are each independently an acyl group (long acyl group) of 7 to 13 carbon atoms, an acyl group (short acyl group) of 2 to 4 carbon atoms, or a ring acyl group selected from a naphthenic acyl group of 6 to 11 carbon atom and an aromatic acyl group of 6 to 11 carbon atoms.

A second aspect of the present invention provides a polyvinyl chloride resin composition, comprising the ester plasticizer in an amount of 10 ~ 100 phr based on a polyvinyl chloride resin.

### Advantageous Effects

When a polyvinyl chloride resin is manufactured using the ester plasticizer using glycerol according to the present invention, there are advantages in that a product having excellent plasticizing efficiency can be obtained, and in that the physical properties such as tensile strength and the like of the product are improved.

### Best Mode

Hereinafter, an ester plasticizer and a polyvinyl chloride resin composition comprising the ester plasticizer according to the present invention will be described in detail.

The ester plasticizer of the present invention, prepared by reacting glycerol with at least one kind of carboxylic acid, is an ester compound represented by Formula 1 below: 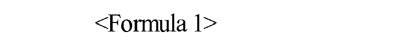 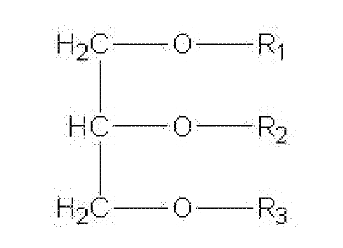 wherein R₁, R₂ and R₃ are each independently an acyl group (long acyl group) of 7 to 13 carbon atoms, an acyl group (short acyl group) of 2 to 4 carbon atoms, or a ring acyl group selected from a naphthenic acyl group of 6 to 11 carbon atom and an aromatic acyl group of 6 to 11 carbon atoms.

The ester plasticizer of the present invention is an ester compound using a basic structure of glycerol, and the formation of the acyl group is determined by carboxylic acid reacting with glycerol. The efficiency of the ester plasticizer is greatly changed according to the kind of carboxylic acid added to form the acyl group. In relation to the efficiency of the ester plasticizer, it can be seen from the following Examples and Comparative Examples that the efficiency of the ester plasticizer is greatly lowered when carboxylic acid of 2 to 4 carbon atoms is not used. Therefore, in the ester plasticizer represented by Formula 1 above, it is preferred that at least one of R₁, R₂ and R₃ be an acyl group of 2 to 4 carbon atoms.

The ester plasticizer of the present invention is prepared by the esterification reaction of glycerol with at least one kind of carboxylic acid. As the carboxylic acid, carboxylic acid of 2 to 4 carbon atoms, such as acetic acid, propionic acid or butyric acid, may be essentially used. In addition, as the carboxylic acid, long-chain carboxylic acid of 7 to 13 carbon atoms, or ring-shaped or aromatic carboxylic acid of 6 to 11 carbon atom, such as benzoic acid, may be preferably used. The molar ratio of the carboxylic acid to glycerol is 1: 0.2 - 3, preferably 1: 0.9 - 2.0. This molar ratio thereof is determined based on the hydroxy group existing in glycerol. It is preferred that an acid catalyst, for example, sodium bisulfate be used in the esterification reaction. Further, p-toluenesulfonic acid, sulfuric acid or the like can be used as the catalyst in the esterification reaction. The catalyst may be used in an amount of z 5 wt% based on a reaction mixture.

Meanwhile, examples of the solvents usable in the esterification reaction include hexane, cyclohexane, toluene and xylene. It is preferred that the esterification reaction be conducted at 100 ~ 160□.

After the esterification reaction, unreacted organic acid and acid catalyst are neutralized by the addition of an alkali reagent such as an aqueous sodium carbonate solution or an aqueous calcium carbonate solution. The coarse ester obtained after phase separation is washed with water, dewatered and then filtered to obtain an object.

The ester plasticizer of the present invention may be any one selected from the group consisting of compounds represented by Formulae 2 to 4 below: 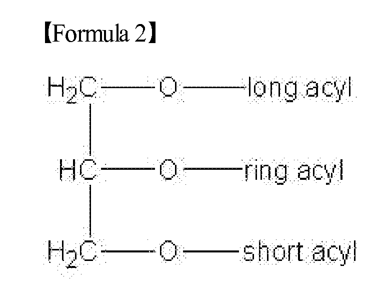 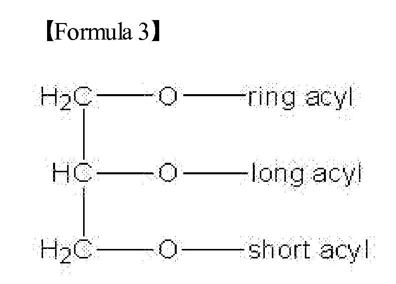 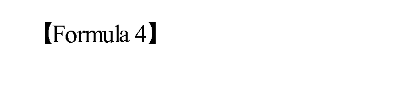 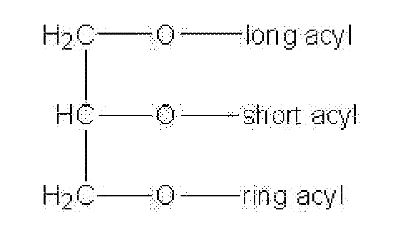 wherein long acyl is an acyl group of 7 to 13 carbon atoms, short acyl is an acyl group of 2 to 4 carbon atoms, and ring acyl is a ring acyl group selected from a naphthenic acyl group of 6 to 11 carbon atom and an aromatic acyl group of 6 to 11 carbon atoms.

The ester plasticizer of the present invention is suitably used together with a polyvinyl chloride resin. The polyvinyl chloride resin is not limited to polyvinyl chloride. Examples of the polyvinyl chloride resin may include: chlorine-containing resins, such as chlorinated polyvinyl chloride, polyvinylidene chloride, chlorinated polyethylene, polyvinyl chloride acetate copolymer, polyvinyl chloride ethylene copolymer, polyvinyl chloride propylene copolymer, polyvinyl chloride styrene copolymer, polyvinyl chloride isobutylene copolymer, polyvinyl chloride vinylidene copolymer, polyvinyl chloride ether copolymers, and blends thereof; and blends, block copolymers and graft copolymers of the chlorine-containing resins and resins containing no chlorine such as acylonitrile-styrene copolymer, acrylonitrile-styrene-butadiene terpolymer, ethylene-vinyl acetate copolymer, polyesters, etc.

The polyvinyl chloride resin composition of the present invention may include the ester plasticizer in an amount of 10 ~ 100 phr based on a polyvinyl chloride resin. The amount of the ester plasticizer of the present invention may be suitably adjusted according to the use of polyvinyl chloride resin composition. When the amount of the e ster plasticizer is less than 10 phr, flexibility or workability, which can be exhibited by a plasticizer, cannot be realized. Further, when the amount thereof is more than 100 phr, it is difficult to ensure necessary mechanical properties, and it is probable that the polyvinyl chloride resin composition will elute.

Meanwhile, the polyvinyl chloride resin composition may further include additives, excluding filler and pigment, in an amount of 0 - 30 phr based on the polyvinyl chloride resin.

Here, examples of the additives, which are general additives that can be selectively included in the polyvinyl chloride resin composition, may include an insulation improver, various kinds of metal salts, polyols, epoxy compounds, a phenolic or sulfuric antioxidant, an ultraviolet absorber, a hindered amine-based photostabilizer, an inorganic stabilizer, an antifogging agent, an anti-misting agent, an auxiliary stabilizer, organic tin compounds, and the like.

Unlike the general additives, filler and pigment can be included in an amount of about 200 phr based on the polyvinyl chloride resin. When the amount of the filler and pigment is more than 200 phr, the density, hardness or flexibility of the polyvinyl chloride resin composition is negatively influenced. Examples of the filler may include calcium carbonate, silica, clay, glass beads, mica, sericite, glass flakes, asbestos, wollastonite, potassium titanate, polarization-maintaining fiber (PMF), gypsum fiber, xonotlite, metal-oxide semiconductor (MOS), phosphate fiber, glass fiber, carbon fiber, aramid fiber, and the like.

The polyvinyl chloride resin composition including the ester plasticizer of the present invention can be used in building materials, such as wall-finishing materials, floor materials, window sashes, wallpaper, and the like; wire covering materials; interior and exterior materials for automobiles; agricultural materials, such as materials for vinyl houses, tunnels and the like; food wrappers; film-forming agents, such as underbody sealant, plastisol, paint, ink and the like; and miscellaneous goods, such as synthetic leather, coated fabrics, hoses, pipes, sheets, toys for infants, gloves and the like. However, the present invention is not limited thereto.

Methods of preparing the polyvinyl chloride resin composition using the ester plasticizer are not particularly limited, and are well known in the related field.

### Mode for Invention

Hereinafter, the present invention will be described in more detail with reference to the following Examples. However, these Examples are set forth to illustrate the present invention, and the scope of the present invention is not limited thereto. In these Examples, the physical properties of samples were evaluated by the following method.

### Hardness

Based on the ASTM D2240, the needle of a hardness tester (A type) was completely pressed onto one point of a sample for 5 seconds, and then the hardness of the sample was measured. Hardness tests were conducted at three points of each sample, and then the average value thereof was obtained. Hardness is used as an index for representing plasticizing efficiency.

### Tensile strength, elongation, elastic modulus at 100% elongation

The tensile strength and elastic modulus of a sample were measured using UTM based on the ASTM D412 method. The tensile strength and elastic modulus thereof were measured at the cut point of a dumbbell-shaped sample after it was pulled at a crosshead speed of 200 mm/min. The elastic modulus at 100% elongation corresponds to the tensile strength at 100% elongation, and is closely related to plasticizing efficiency.

### Maximum torque

The maximum torque occurring at the time of mixing a polyvinyl chloride with a plasticizer was measured using a Brabender Tester.

### [Example 1]

### Preparation of an ester plasticizer using glycerol, benzoic acid, octanoic acid and acetic acid

First, 1.0 mol of glycerol, 0.7 mol of benzoic acid, 0.7 mol of octanoic acid, 0.7 mol of acetic acid, 200 g of toluene as a solvent, and 3.0 g of sodium bisulfate as a catalyst were put into a 2L round flask provided with a stirrer and a condenser, and were then heated to 130°C to conduct a reaction for 12 hours.

After the reaction, unreacted organic acid were depressurized to 5 mmHg at 200□ by a vacuum pump, neutralized by 100 wt% of an aqueous sodium carbonate solution, water-washed, dewatered and then filtered by an adsorbent to obtain an triglyceride-type ester plasticizer represented by Formula 1 above.

### Preparation of a polyvinyl chloride resin composition

Test samples were fabricated in order to evaluate the performance of the obtained ester plasticizer. That is, 50 phr of the obtained ester plasticizer and 1 phr of a stabilizer (LFX-1100) were mixed with a polyvinyl chloride resin (LS-100, manufactured by LG Chemicals Co., Ltd.) 100 parts by weight, and then the mixture was preheated to 185 °C for 1 minute, pressurized for 1.5 minutes and cooled for 2 minutes to obtain a sheet having a thickness of 2 mm. The sheet was formed into various dumbbell-shaped test samples.

The above-mentioned tests were conducted using these test samples, and the results thereof are given in Table 1 below.

### [Example 2]

An ester plasticizer and a polyvinyl chloride resin composition were prepared in the same manner as Example 1, except that decanoic acid was used instead of octanoic acid. The test results thereof are given in Table 1 below.

### [Example 3]

An ester plasticizer and a polyvinyl chloride resin composition were prepared in the same manner as Example 1, except that 2-ethyl hexanoic acid was used instead of octanoic acid. The test results thereof are given in Table 1 below.

### [Example 4]

An ester plasticizer and a polyvinyl chloride resin composition were prepared in the same manner as Example 1, except that naphthenic acid was used instead of octanoic acid. The test results thereof are given in Table 1 below.

### [Comparative Example 1]

An ester plasticizer and a polyvinyl chloride resin composition were prepared in the same manner as Example 1 using only 1.0 mol of glycerol, 0.7 mol of benzoic acid and 0.7 mol of octanoic acid. The test results thereof are given in Table 1 below.

### [Comparative Example 2]

A test sample was fabricated in the same manner as Example 1 using di-2-ethylhexyl phthalate as a plasticizer. The same test that had been conducted in Example 1 was conducted using this test sample, and the results thereof are given in Table 1 below.

### [Comparative Example 3]

A test sample was fabricated in the same manner as Example 1 using diisononyl phthalate as a plasticizer instead of the di-2-ethylhexyl phthalate of Comparative Example 2. The same test that had been conducted in Example 1 was conducted using this test sample, and the results thereof are given in Table 1 below.

### [Comparative Example 4]

A test sample was fabricated in the same manner as Example 1 using trioctyl trimellitate as a plasticizer. The same test that had been conducted in Example 1 was conducted using this test sample, and the results thereof are given in Table 1 below.

**[Table 1]**

| Measured items | Ex.1 | Ex. 2 | Ex. 3 | Ex. 4 | Co. Ex.1 | Co. Ex. 2 | Co. Ex. 3 | Co. Ex. 4 |
|---|---|---|---|---|---|---|---|---|
| Hardness (ShoreA) | 76 | 78 | 77 | 80 | 97 | 79 | 81 | 87 |
| Tensile strength (Kgf/cm2) | 208 | 218 | 210 | 201 | 240 | 197 | 197 | 214 |
| Elongation (%) | 396 | 405 | 387 | 362 | 302 | 359 | 343 | 353 |
| Modulus (Kgf/cm2) | 77 | 82 | 79 | 85 | 158 | 84 | 98 | 118 |
| Maximum torque (Nm) | 4.3 | 4.1 | 4.4 | 4.0 | 1.2 | 4.4 | 4.5 | 4.4 |

From the results of Table 1 above, it can be presumed that the plasticizing efficiency of the plasticizers of Examples 1, 2, 3 and 4 is higher than that of the plasticizers of Comparative Examples 2, 3 and 4, and that the physical properties, such as tensile strength, elongation and the like, of the plasticizers of Examples 1, 2, 3 and 4 are equal to or greater than those of the plasticizers of Comparative Examples 2, 3 and 4. Meanwhile, it was found that the plasticizing efficiency of the plasticizer of Comparative Example 1, which was prepared without using carboxylic acid of 2 to 4 carbon atoms, is very low compared to that of the plasticizers of Examples 1, 2, 3 and 4. Therefore, it is expected that, since the novel plasticizer of the present invention has high plasticizing efficiency, it can be formed in various shapes, and thus it can be variously utilized.

As described above, although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. An ester plasticizer, represented by Formula 1 below: 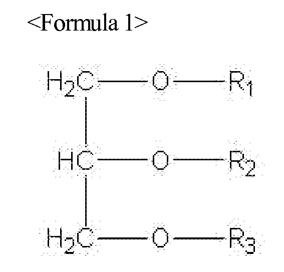 wherein R₁, R₂ and R₃ are each independently an acyl group (long acyl group) of 7 to 13 carbon atoms, an acyl group (short acyl group) of 2 to 4 carbon atoms, or a ring acyl group selected from a naphthenic acyl group of 6 to 11 carbon atom and an aromatic acyl group of 6 to 11 carbon atoms.

2. The ester plasticizer according to claim 1, wherein at least one of R₁, R₂ and R₃ is an acyl group of 2 to 4 carbon atoms.

3. The ester plasticizer according to claim 1, wherein the ester plasticizer is selected from the group consisting of compounds represented by Formulae 2 to 4 below: 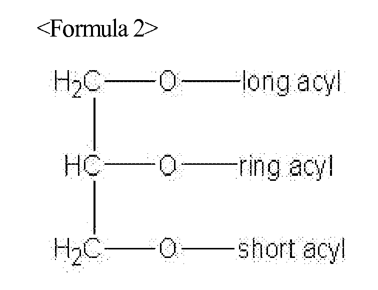 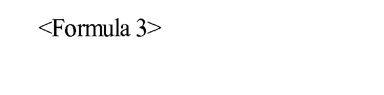 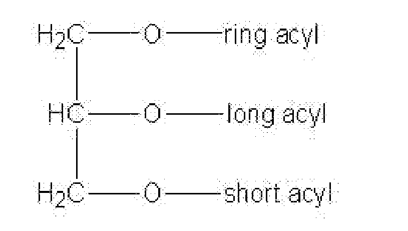 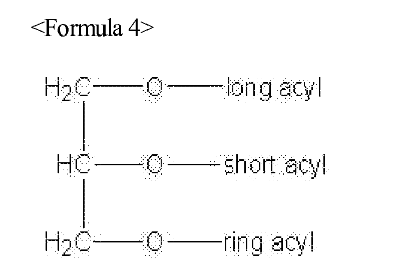 wherein long acyl is an acyl group of 7 to 13 carbon atoms, short acyl is an acyl group of 2 to 4 carbon atoms, and ring acyl is a ring acyl group selected from a naphthenic acyl group of 6 to 11 carbon atom and an aromatic acyl group of 6 to 11 carbon atoms.

4. The ester plasticizer according to claim 1, wherein the ester plasticizer is prepared by reacting glycerol with at least one kind of carboxylic acid.

5. The ester plasticizer according to claim 4, wherein the carboxylic acid is selected from the group consisting of acetic acid, propionic acid, and butyric acid.

6. A polyvinyl chloride resin composition, comprising the ester plasticizer of any one of claims 1 to 5 in an amount of 10 ~ 100 phr based on a polyvinyl chloride resin.

7. The polyvinyl chloride resin composition according to claim 6, further comprising additives, excluding filler and pigment, in an amount of 0 - 30 phr based on a polyvinyl chloride resin.
